# EUROPÄISCHE PATENTSCHRIFT

(11) **EP 1 871 423 B1**
(45) Veröffentlichungstag und Bekanntmachung des Hinweises auf die Patenterteilung: **01.08.2012**
(21) Anmeldenummer: 06742238.6
(22) Anmeldetag: 12.04.2006
(51) Int. Cl.: A61K 47/48, A61K 9/14

(54) **NANOPARTIKEL-WIRKSTOFF-KONJUGATE**
NANOPARTICLE/ACTIVE INGREDIENT CONJUGATE
CONJUGUES DE NANOPARTICULES ET D'AGENTS ACTIFS

(30) Priorität: 12.04.2005 DE 102005016873; 27.04.2005 US 675100 P
(43) Veröffentlichungstag der Anmeldung: 02.01.2008
(73) Patentinhaber: MagForce AG, 10589 Berlin (DE)
(72) Erfinder: JORDAN, Andreas, 14129 Berlin (DE); WALDOEFNER, Norbert, 47167 Duisburg (DE); DECKEN, Klaus, 12159 Berlin (DE); SCHOLZ, Regina, 13125 Berlin (DE)
(74) Vertreter: Bösl, Raphael Konrad
(86) Internationale Anmeldenummer: PCT/DE2006/000653
(87) Internationale Veröffentlichungsnummer: WO 2006/108405

(56) Entgegenhaltungen:
- WO-A-03/026618
- WO-A2-02/43708
- WO-A2-2005/042142
- WO-A2-2005/065282
- WO-A2-2005/070471
- DE-A1- 4 428 851
- US-A- 5 411 730

## Beschreibung

Die vorliegende Erfindung betrifft Nanopartikel, an die therapeutisch wirksame Substanzen gebunden sind, wobei die Freisetzung der therapeutisch wirksamen Substanzen durch ein magnetisches Wechselfeld bewirkt, initiiert oder wesentlich gesteigert wird.

Es ist bekannt, dass superparamagnetische Nanopartikel als Wirkstoffträger zur Behandlung von Krankheiten eingesetzt werden können. Dabei werden verschiedene Ansätze verfolgt. Eine bekannte Strategie basiert z.B. auf dem so genannten "Magnetic Drug-Targeting", bei dem versucht wird, eine lokale Konzentrationserhöhung der Wirkstoffe mittels eines Magnetfeldes zu erreichen (DE 10059151 A, Alexiou). Ebenso wird versucht, den Partikeln auf chemischem Wege Zielfindungseigenschaften zu verleihen, um so eine Anreicherung in bestimmten Körperregionen zu erreichen (DE 4428851 A1, EP 0516252 A2). Mehrschalige Teilchen zur Einschleusung von Partikel-Wirkstoff-Konjugaten in Tumorzellen werden in der Patentschrift WO 98/58673 (INM) beschrieben.

WO 2005/070471 A2 offenbart magnetische, therapeutische Partikel umfassend magnetisches Material und bioaktive Mittel.

In WO 02/43708 A2 werden magnetische Partikel zur zielgerichteten regionalen Therapie von Krankheiten beim Menschen beschrieben.

Das US-Patent Nr. 5, 411, 730 offenbart superparamagnetische Partikel zur medizinischen Anwendung einschließlich hyperthermalen Techniken, lokalem Erhitzen und der gewebespezifischen Freisetzung therapeutischer Mittel.

Aus WO 2005/042142 sind thermosensitive, biokompartible Polymerträger mit veränderbarer physikalischer Struktur für die Therapie, Diagnostik und Analytik bekannt.

WO 03/026618 A1 beschreibt nicht-invasive Verfahren zur gezielten Lokalisierung biologisch aktiver Moleküle umfassend thermosensitive Partikel mit darin eingeschlossenen Molekülen, die dem Patienten verabreicht werden und aus denen die Moleküle unter Verwendung einer Energiequelle freigesetzt werden.

Aus WO 2004/0655282 A2 ist die Verwendung von magnetischen Partikeln in Verbindung mit Magnetfeldern zur Behandlung von Krankheiten wie beispielsweise Krebs bekannt.

Aufgabe der vorliegenden Erfindung ist es, Nanopartikel derart mit therapeutisch wirksamen Substanzen zu beladen, dass im gesunden Gewebe keine nennenswerte Freisetzung der therapeutisch wirksamen Substanzen stattfindet und nach dem Eintreten der Nanopartikel in das Tumorgewebe und in die Tumorzellen eine gesteuerte Freisetzung der therapeutisch wirksamen Substanzen erfolgen kann.

Die Aufgabe wird durch die Nanopartikel gemäß Anspruch 1 sowie die pharmazeutische Zusammensetzung gemäß Anspruch 12 und die Verwendung der Nanopartikel gemäß Anspruch 13 gelöst.

Weitere vorteilhafte Ausgestaltungen ergeben sich aus den abhängigen Ansprüchen, den Beispielen und der Beschreibung.

Die vorliegende Erfindung betrifft Nanopartikel, wobei an das Nanopartikel und wobei die Ablösung der mindestens einen therapeutisch wirksamen Substanz von dem Nanopartikel durch ein magnetisches Wechselfeld bewirkt, initiiert oder wesentlich gesteigert wird. Dabei wird die mindestens eine therapeutisch wirksame Substanz über ein Linker-Molekül gebunden ist, wobei das Linker-Molekül eine thermolabile, elektro-magneto-labile, photolabile, säurelabile, interkalierbare oder enzymatisch spaltbare Gruppe enthält und wobei das Linker-Molekül ein Nukleinsäuremolekül, ein Polypeptid, eine Peptid-Nukleinsäure, ein Aptamer, DNA, RNA, ein Leucin-Zipper, ein Oligonukleotid, ein Oligopeptid, eine Hapten-Antikörper-Brücke oder eine Biotin-Avidin-Brücke ist, oder wobei im Linker-Molekül eine Bindung von Biotin an Avidin oder Streptavidin vorliegt,

mindestens eine therapeutisch wirksame Substanz durch den direkten Einfluß des alternierenden magnetischen Feldes oder durch die lokale Erwärmung aufgrund des alternierenden magnetischen Feldes freigesetzt. Die Freisetzung geschieht bevorzugt dadurch, dass ein thermisch labiler Linker zwischen dem Wirkstoff, d.h. der therapeutisch wirksamen Substanz und dem Nanopartikel thermisch gespalten wird und/oder ein Linker verwendet wird, der gegenüber einem alternierenden magnetischen Feld labil ist. Die vorliegende Erfindung besteht also darin, eine therapeutisch wirksame Substanz, insbesondere ein Cytostatikum über einen thermisch und/oder durch ein magnetisches Feld spaltbaren Linker an ein Nanopatikel zu binden.

Die erfindungsgemäßen Nanopartikel sind dadurch charakterisiert, dass an das Nanopartikel mindestens eine therapeutisch wirksame Substanz über ein Linker―Molekül gebunden ist und wobei die Ablösung der mindestens einen therapeutisch wirksamen Substanz von dem Nanopartikel durch ein magnetisches Wechselfeld bewirkt oder initiiert oder wesentlich gesteigert wird.

Anders gesagt betrifft die vorliegende Erfindung Nanopartikel, wobei an das Nanopartikel mindestens eine therapeutisch wirksame Substanz kovalent oder ionisch oder über Wasserstoffbrücken oder über Komplexverbindung oder über Interkalation oder über lipophile Wechselwirkungen mittels eines Linkers gebunden ist und der Linker thermisch initiiert und/oder initiiert durch ein elektromagnetisches bzw. magnetisches Feld gespalten werden kann.

Thermisch initiierte Spaltung bedeutet, dass eine lokale Erwärmung auf über 45°C bevorzugt über 50°C unter physiologischen Bedingungen ausreicht, um den Linker zu spalten. Eine Spaltung initiiert durch ein elektromagnetisches bzw. magnetisches Feld bedeutet, dass unter physiologischen Bedingungen das Anlegen eines elektromagnetischen bzw. magnetischen Feldes eine Spaltung des Linkers auslöst, sei es nur durch das elektromagnetische bzw. magnetische Feld und/oder durch eine lokale pH-Wert Erniedrigung, welche durch das elektromagnetische bzw. magnetische Feld hervorgerufen wird.

Die Anbindung der mindestens einen therapeutisch wirksamen Substanz, d.h. der Moleküle mindestens einer therapeutisch wirksamen Substanzklasse oder eines bestimmten Wirkstoffs erfolgt vorzugsweise kovalent oder durch eine überwiegend kovalente Bindung und/oder durch eine ausreichend starke ionische Bindung, Einlagerungsverbindung oder Komplexbindung, bzw. eine Anordnung einer ausreichenden Anzahl von Wasserstoffbrückenbindungen oder hydrophober Ein wechselndes Magnetfeld wirkt als externe Anregung, die im Falle von superparamagnetischen Partikeln verschiedene Relaxationsprozesse der Partikel in Gang setzt. Diese Prozesse führen u.A. zu einer Erwärmung der Partikel und ihrer Umgebung. Diese durch das magnetische Wechselfeld in Gang gesetzten Prozesse werden erfindungsgemäß dazu benutzt, die Bindung zwischen Nanopartikel und therapeutisch wirksamer Substanz zu spalten oder die Spaltung stark zu beschleunigen. Dabei kann z.B. die Geschwindigkeit der Abspaltung durch biologische Prozesse (z.B. enzymatisch) durch den Impuls stark erhöht werden, so dass erst nach eingeschaltetem Impuls eine nachhaltige Konzentrationserhöhung des Wirkstoffs am Zielort erreicht werden kann. Ebenso kann die Bindung so konstruiert sein, dass eine Abspaltung durch chemische Reaktionen (z.B. Hydrolyse) in Gang gesetzt oder merklich beschleunigt wird. Darüber hinaus kann durch die Magnetfeld-induzierte Erwärmung ein Aufschmelzen eines als Linker eingesetzten Nukleinsäure- oder Polypeptid-Moleküls erfolgen.

Die therapeutisch wirksamen Substanzen werden über ein Linker-Molekül gebunden. Das Linker-Molekül wird vorzugsweise mittels einer Amidbindung oder Esterbindung an die Nanopartikel bzw. an das jeweilige Nanopartikel gebunden.

Als Linker können erfindungsgemäß auch Nukleinsäuren (Desoxyribonukleinsäuren (DNA), Ribonukleinsäure (RNA) oder Peptid-Nukleinsäuren (PNA)) oder Polypeptide verschiedener Länge eingesetzt werden. Die erforderlichen Moleküle können wahlweise gentechnisch oder synthetisch hergestellt werden. Die Linker können thermisch induziert, magnetisch induziert oder säureinduziert unter physiologischen Bedingungen gespalten werden.

Spaltung des Linkers bedeutet, dass der Linker zumindest eine Bindung innerhalb des Linkers enthält, welche durch Wärmeeinwirkung, Einwirkung eines magnetischen Feldes, d.h. magnetischen Impulses oder durch Säureeinwirkung unter physiologischen Bedingungen gespalten werden kann. Die Spaltung dieser Bindung sollte durch die Einwirkung von Wärme (bevorzugt mindestes 45°C) und/oder des magnetischen Feldes und/oder von Säure unter physiologischen Bedingungen mindestens doppelt so schnell verlaufen als ohne diese Einwirkung. Die Bildung von Säure und die Absenkung des lokalen pH-Wertes kann z.B. durch bereits abgetötete Zellen verursacht werden. Der Begriff "Bindung innerhalb des Linkers" umfasst auch die Bindung des Linkers zum Nanopartikel sowie die Bindung des Linkers zur therapeutisch wirksamen Substanz. Zudem kann der Linker auch aus zwei oder drei Linkermolekülen zusammengesetzt sein.

Aminosilane oder eine Aminogruppen-tragende Hülle bzw. Beschichtung kann eine Ankopplung der Wirkstoffe z.B. an oberflächennahe Aminogruppen erfolgen. Hierbei kann eine Ankopplung z.B. über Succinimidyl-Ester, Sulfosuccinimidyl-Ester, Isothiocyanate, Triazinyl-Chloride, Sulfonyl-Chloride Tetrafluorphenyl-Ester oder auch über Aldehyd-Gruppen erfolgen. Voraussetzung ist, dass der Wirkstoff auf chemischem Wege mit solchen Gruppen versehen werden kann. Sollte eine direkte Ankopplung des Wirkstoffs über diese Methoden nicht möglich sein, ist der Einsatz eines "Linker-Moleküls" möglich. Dieser "Linker" verbindet den Wirkstoff mit den funktionellen Gruppen der Schutzhülle und bietet somit eine erhöhte Variabilität der Kopplungsmöglichkeiten. Erfindungsgemäß enthält das Linker-Molekül eine thermolabile, elektro-magneto-labile, photolabile, säurelabile, interkalierbare, interkalierte oder enzymatisch spaltbare Gruppe. Darüber hinaus kann auch der Freisetzungsmechanismus über den Linker gesteuert werden. So kann der Linker auch Gruppen einführen, die eine Abspaltung der Wirkstoffe ermöglichen. In Frage kommen z.B. pH-spaltbare Acetal-, Ester-, Hydrazon- oder Imin-Gruppen. Ebenso sind Peptid-Sequenzen als Linker geeignet, bei denen erst eine enzymatische Abspaltung oder ein Aufschmelzen einer nicht-kovalenten Bindung den Wirkstoff freisetzt. Darüber hinaus kommen DNA, RNA und PNA-Moleküle als vorzugsweise doppelsträngige Linker in Frage, wobei die Freisetzung durch eine thermisch induzierte Aufschmelzung der Doppelstränge erfolgt.

Erfindungsgemäß dürfen aber nur Linker eingesetzt werden, die unter normalen physiologischen Bedingungen keine, oder nur langsame Abspaltungsraten bewirken. Die Linker-Moleküle können z.B. so konstruiert sein, dass eine Freisetzung im Zielgebiet (z.B. enzymatisch in der Tumorzelle) zwar möglich ist, dieser unter normalen Bedingungen aber so langsam ist, dass keine therapeutische Konzentration des Wirkstoffs erreicht werden kann. Erst durch den äußeren Impuls des magnetischen Wechselfeldes setzt die Abspaltung des Linker-Moleküls bzw. die Spaltung des Linker-Moleküls in ausreichender Geschwindigkeit ein und führt zu einer Aktivierung des Wirkstoffs. Das wird vorzugsweise dadurch erreicht, dass die verwendeten Linker erst nach einem thermisch induzierten Aufschmelzen von Nukleinsäure-Doppelsträngen bzw. Mehrfachsträngen oder alternativ von Peptid-Dimeren bzw. Peptid-Oligomeren eine Konformation einnehmen, die eine enzymatische Spaltung ermöglicht.

Ebenso wie im Falle von Aminosilan-stabilisierten Partikeln kann mit anderen, durch verschiedenartige funktionelle Gruppen (z.B. Carboxy, Epoxy, Aldehyd) stabilisierte magnetische Partikel verfahren werden. Entscheidend ist, dass die Kopplungsmethode so gewählt wird, dass eine Freisetzung nur unter den o.g. Wechselwirkungen, so dass eine unkontrollierte Freisetzung von therapeutisch wirksamer Substanz weitgehend unterbleibt. Als unkontrollierte Freisetzung wird die Ablösung von therapeutisch wirksamer Substanz im gesunden Gewebe verstanden, insbesondere die Ablösung ohne die Einwirkung eines magnetischen Wechselfeldes.

Eine solche unkontrollierte Freisetzung führt dazu, dass therapeutisch wirksame Substanzen dort freigesetzt werden, wo sie eher schädliche Nebenwirkungen als therapeutische Effekte bewirken, nämlich außerhalb des kanzerogenen Gewebes bzw. der Tumorzellen.

Die therapeutisch wirksamen Substanzen bleiben somit fest an die Nanopartikel gebunden und werden samt der Nanopartikel in die Krebszellen transportiert. Auf dem Weg der Nanopartikel in die Krebszellen wird nur eine geringe Menge bis hin zu einer unwesentlichen Menge der therapeutisch wirksamen Substanzen freigesetzt. Angekommen in den Krebszellen erfolgt dann die Freisetzung der therapeutisch wirksamen Substanzen mittels eines magnetischen Wechselfeldes, insbesondere eines äußeren bzw. von außen angelegten magnetischen Wechselfeldes (Impuls).

In diesem Zusammenhang bedeutet "durch ein magnetisches Wechselfeld bewirkt oder initiiert", dass zum einen das magnetische Wechselfeld bzw. die Impulse direkt die Freisetzung bzw. Ablösung bewirken oder indirekt beispielsweise über die Aktivierung bzw. Induktion der Genexpression von Enzymen oder die Erzeugung von Wärme.

Die Nanopartikel selbst bestehen aus einem magnetischen Material, vorzugsweise einem ferromagnetischen, antiferromagnetischen, ferrimagnetischen, antiferrimagnetischen oder superparamagnetischen Material, weiter bevorzugt aus Eisenoxiden, insbesondere superparamagnetischen Eisenoxiden oder aus reinem Eisen, welches mit einer Oxidschicht versehen ist. Derartige Nanopartikel können durch ein magnetisches Wechselfeld erwärmt werden. Eine Erwärmung des die Nanopartikel enthaltenden Gewebes auf über 50°C ist möglich. Derartig hohe Temperaturen können erreicht werden, da bis zu 800 pg und mehr Eisen in Form der Nanopartikel pro Tumorzelle aufgenommen werden können.

Die Nanopartikel bestehen vorzugsweise aus Eisenoxiden und insbesondere aus Magnetit (Fe₃O₄), Maghemit (γ-Fe₂O₃) oder Mischungen dieser beiden Oxide. Allgemein können die bevorzugten Nanopartikel durch die Formel FeOₓ wiedergegeben werden, worin X eine Zahl von 1 bis 2 bedeutet. Die Nanopartikel weisen vorzugsweise einen Durchmesser von weniger als 500 nm auf. Vorzugsweise besitzen die Nanopartikel einen durchschnittlichen Durchmesser von 15 nm oder liegen vorzugsweise in dem Größenbereich von 1 - 100 nm und insbesondere bevorzugt im Bereich von 10 - 20 nm.

Neben den magnetischen Materialien der Formel FeOₓ, worin X eine Zahl im Bereich von 1,0 bis 2,0 ist, sind erfindungsgemäß auch Materialien der allgemeinen Formel MFe₂O₄ mit M = Co, Ni, Mn, Zn, Cd, Ba oder andere Ferrite einsetzbar. Ferner eignen sich auch Silica- oder Polymerpartikel, in die magnetische Materialien wie beispielsweise die hierin genannten magnetischen Materialien eingelagert und/oder angebunden sind.

An diese Nanopartikel, insbesondere superparamagnetische Nanopartikel werden nun therapeutisch wirksame Substanzen gebunden, wobei eine kovalente Bindung bevorzugt ist. Als therapeutisch wirksame Substanzen können anti-proliferative, anti-migrative, anti-angiogene, anti-thrombotische, antiinflammatorische, antiphlogistische, zytostatische, zytotoxische, anti-koagulative, anti-bakterielle, anti-virale und/oder anti-mykotische Wirkstoffe gewählt werden, wobei anti-proliferative, anti-migrative, anti-angiogene, zytostatische und/oder zytotoxische Wirkstoffe sowie Nukleinsäuren, Aminosäuren, Peptide, Proteine, Kohlenhydrate, Lipide, Glycoproteine, Glycane oder Lipoproteine mit anti-proliferativen, anti-migrativen, antiangiogenen, anti-thrombotischen, antiinflammatorischen, antiphlogistischen, zytostatischen, zytotoxischen, anti-koagulativen, anti-bakteriellen, anti-viralen und/oder anti-mykotischen Eigenschaften bevorzugt sind. Darüberhinaus können diese Substanzen auch Radiosensitizer oder Sensitizer oder Verstärker anderer auch kombinierter konventioneller Krebsbehandlungsmethoden sein oder solche Sensitizer enthalten.

Als zytotoxische und/oder zytostatische Verbindungen, d.h. chemische Verbindungen mit zytotoxischen und/oder zytostatischen Eigenschaften können unter anderem Alkylierungsmittel, Antibiotika mit zytostatischen Eigenschaften, Antimetabolite, Mikrotubuli-Inhibitoren und Topoisomerase-Inhibitoren, Platin-enthaltende Verbindungen und andere Zytostatika wie beispielsweise Asparaginase, Tretinoin, Alkaloide, Podophyllotoxine, Taxane und Miltefosin^{®}, Hormone, Immunmodulatoren, monoklonale Antikörper, Signaltransduktoren (Signaltransduktionsmoleküle) und Zytokine eingesetzt werden.

Als Beispiele für Alkylierungsmittel können unter anderem Chlorethamin, Cyclophosphamid, Trofosfamide, Ifosfamid, Melphalan, Chlorambucil, Busulfan, Thiotepa, Carmustin, Lomustin, Dacarbazin, Procarbazin, Temozolomid, Treosulfan, Estramustin und Nimustin genannt werden.

Beispiele für Antibiotika mit zytostatischen Eigenschaften sind Daunorubicin, Doxorubicin (Adriamycin), Dactinomycin, Mitomycin C, Bleomycin, Epirubicin (4-Epi-Adriamycin), Idarubicin, Dactinomycin, Mitoxantron, Amsacrin und Actinomycin D.

Methotrexat, 5-Fluorouracil, 6-Thioguanin, 6-Mercaptopurin, Fludarabin, Cladribin, Pentostatin, Gemcitabin, Cytarabin, Azathioprin, Raltitrexed, Capecitabin, Cytosinarabinosid, Tioguanin und Mercaptopurin können als Beispiele für Antimetabolite (antimetabolische Wirkstoffe) angeführt werden.

Zu der Klasse der Alkaloide und Podophyllotoxine gehören unter anderem Vincristin, Vinblastin, Vindesin, Etoposid als auch Teniposid. Des weiteren können Platin-enthaltende Verbindungen erfindungsgemäß eingesetzt werden. Als Platin-enthaltende Verbindungen seien beispielsweise Cisplatin, Carboplatin und Oxaliplatin genannt. Zu den Mikrotubuli-Inhibitoren zählen beispielsweise Alkaloide wie beispielsweise Vinca-Alkaloide (Vincristin, Vinblastin, Vindesin, Venorelbin) und Paclitaxel (Taxol^{®}) sowie Derivate des Paclitaxel. Als Topoisomerase-Inhibitoren können beispielsweise Etoposid, Teniposid, Camptothecin, Topotecan und Irinotecan genannt werden.

Paclitaxel und Docetaxel sind Beispiele für die Verbindungsklasse der Taxane und zu den anderen zytostatischen Wirkstoffen (anderen Zytostatika) zählen beispielsweise Hydroxycarbamide (Hydroxyurea), Imatinib, Miltefosin^{®}, Amsacrin, Topotecan (Topoisomerase-I-Inhibitor), Pentostatin, Bexaroten, Biolimus A9, Rapamycin (Sirolimus) , Rhodomycin D, Ametantron, Bendamustin, Oxazaphosphorine, 5'-Deoxy-5-fluorouridin, 9-Aminocamptothecin, Podophyllotoxin-Derivate, Mitopodozid, Vinca-Alkaloide, Calicheamicine, Maytansinoide, Tretinoin und Asparaginase. Vertreter der Verbindungsklasse der monoklonalen Antikörper sind unter anderem Trastuzumab (auch bekannt als Herceptin^{®}), Alemtuzumab (auch bekannt als MabCampath^{®}) und Rituximab (auch bekannt als MabThera^{®}).

Erfindungsgemäß können auch Hormone wie beispielsweise Glucocorticoide (Prednison), Oestrogene (Fosfestrol, Estramustin), LHRH (Buserelin, Goserelin, Leuprorelin, Triptorelin), Flutamid, Cyproteronacetat, Tamoxifen, Toremifen, Aminoglutethimid, Formestan, Exemestan, Letrozol und Anastrozol eingesetzt werden. Zu den Klassen der Immunmodulatoren, Zytokine, Antikörper und Signaltransdukctoren zählen Interleukin-2, Interferon-α, Erythropoietin, G-CSf, Trastuzumab (Herceptin^{®}), Rituximab (MabThera^{®}), Efitinib (Iressa^{®}), Ibritumomab (Zevalin^{®}), Levamisol sowie Retinoide.

Die vorgenannten Wirkstoffe werden vorzugsweise kovalent an die Nanopartikel gebunden. Die Anbindung der Wirkstoffe kann beispielsweise über Hydroxygruppen, Aminogruppen, Carbonylgruppen, Thiolgruppen oder Carboxylgruppen erfolgen, je nachdem, welche funktionellen Gruppen der jeweilige Wirkstoff trägt. So kann beispielsweise Doxorubicin über seine primäre Hydroxygruppe als Ester gebunden werde, Platin-Derivate (Cisplatin, Carboplatin, Oxaliplatin ect.) mittels nukleophiler Substitution am Platin an eine Aminogruppe gekoppelt werden, oder Paclitaxel über eine Iminbindung gebunden werden.

Hydroxygruppen werden vorzugsweise als Ester, Acetal oder Ketal gebunden, Thiogruppen vorzugsweise als Thioester, Thioacetal oder Thioketal, Aminogruppen vorzugsweise als Amide und teilweise auch als Imine (Schiffsche Basen) oder durch Reaktion mit einer Isocyanat-Gruppe als Urethan, Carboxylgruppen vorzugsweise als Ester oder Amide und Carbonylgruppen vorzugsweise als Acetale bzw. Ketale.

Die Herstellung von Nanopartikeln, jedoch ohne Wirkstoff und auch ohne Beschichtung ist ausführlich in DE 4428851 A beschrieben. Ferner ist die Funktionalisierung der Oberfläche der Nanopartikel bekannt, so dass nach bekannten Verfahren Aminogruppen, Hydroxygruppen, Carboxylgruppen oder Carbonylgruppen auf der Oberfläche der Nanopartikel erzeugt werden können.

Die vorliegende Erfindung betrifft daher Nanopartikel, welche über eine Vielzahl von Aminogruppen, Hydroxygruppen, Carboxylgruppen oder Carbonylgruppen auf der Oberfläche verfügen, und wobei an zumindest einen Teil dieser funktionellen Gruppen Linker mittels einer Iminbindung, Aminbindung, Esterbindung, Amidbindung oder Ketalbindung gebunden sind und ferner diese Linker die therapeutische wirksame Substanz kovalent, ionisch, komplexiert, lipophhil oder über Wasserstoffbrücken binden.

Ein besonderes Merkmal einer bevorzugten Ausführungsform der erfindungsgemäßen Nanopartikel ist, dass die Wirkstoffe über spezielle Bindungstypen an die magnetischen Nanopartikel gekoppelt sind. Diese Bindungen sind so konstruiert, dass eine Freisetzung der Wirkstoffe durch ein äußeres magnetisches Wechselfeld (Impuls) stimuliert werden kann.

Um die erforderliche Spaltbarkeit zu gewährleisten, weisen die Linker mindestens eine der folgenden funktionellen Gruppen auf: ―S―S―, ―O―P(=O)(O⁻)―O―, ―CO―CO―, ―NH―CO―CO―NH―, ―C=N―C, Ketale, ―CO―NH―N=C―, Trioxysilane (―O―)(―O―)(―O―)Si―C oder Acetale.

Geeignete Linker haben beispielsweise folgende Form:

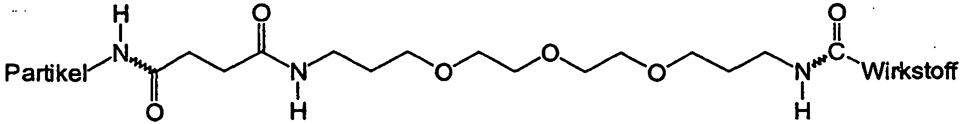

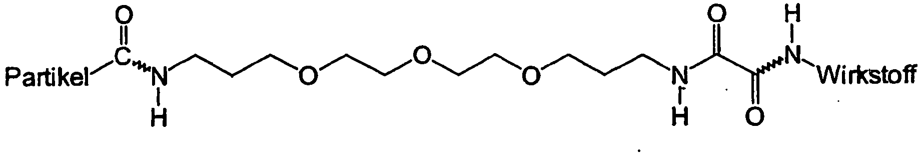

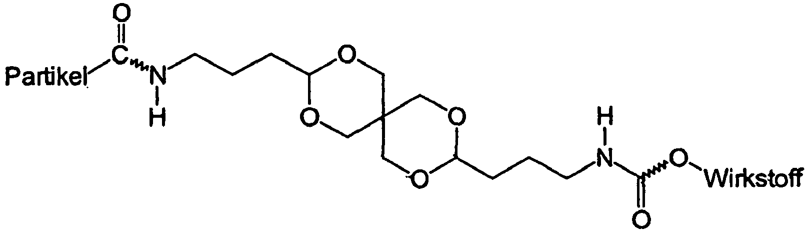

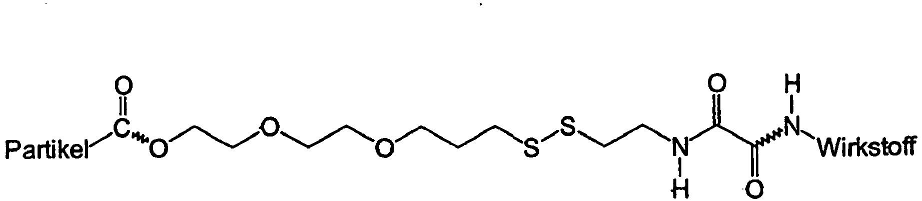

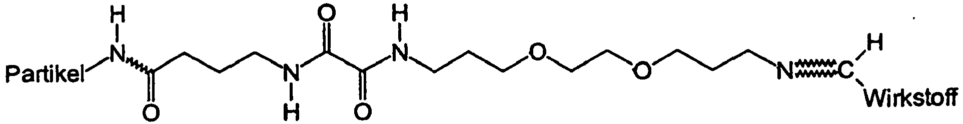

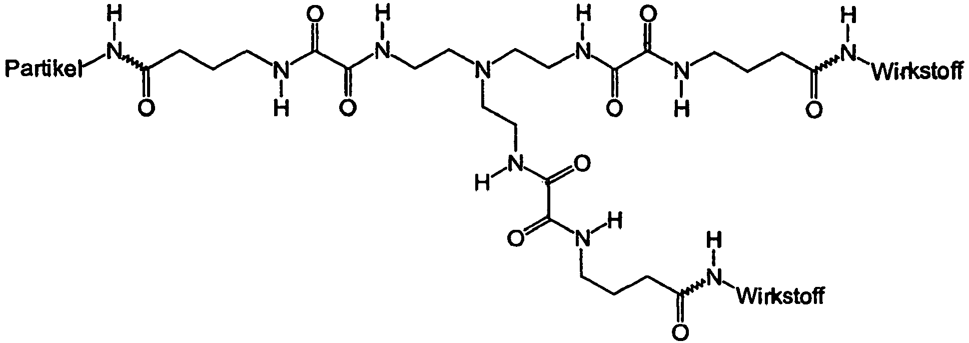

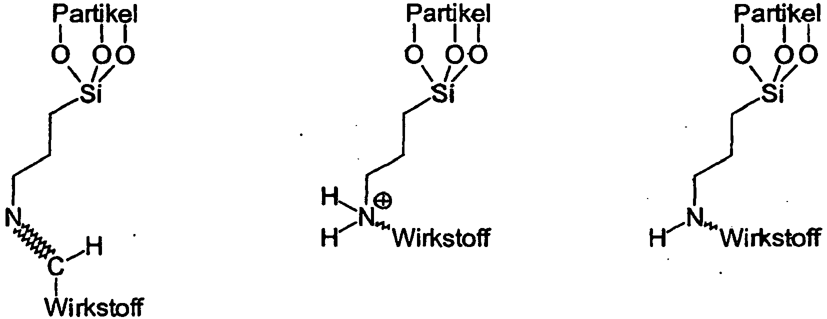

Die Zick-Zack-Linie gibt die Bindung zwischen Wirkstoff und Linker bzw. Linker und Nanopartikel an.

Als Nukleinsäuren werden solche - vorzugsweise doppelsträngigen - Konstrukte bevorzugt, die einen Schmelzpunkt im Bereich von 40-60°C besitzen. Beim Einsatz doppelsträngiger DNA , RNA oder PNA verfügt ein Strang über eine zur Kopplung an die Partikel befähigte Gruppe (z.B. eine Amino- oder Carboxy-Gruppe, die über eine Phosphoramidat-Gruppe angekoppelt wurde). Der komplementäre Strang kann z.B. den Wirkstoff tragen, der ebenfalls über eine kovalente Bindung gekoppelt ist. Durch die Basenpaarung zwischen den Strängen wird so auch der Wirkstoff an die Partikel gekoppelt. Eine Freisetzung des Wirkstoffs kann nun durch das Aufschmelzen der Doppelhelix bei Wärmeentwicklung im magnetischen Wechselfeld erfolgen. Dabei trennen sich die Einzelstränge und die Wirksubstanz wird vom Partikel entkoppelt. Über die Auswahl entsprechender Homo- oder Hetero-Hybride aus DNA-DNA, DNA-RNA, DNA-PNA, RNA-RNA, RNA-PNA oder PNA-PNA lässt sich sowohl der Schmelzpunkt als auch das Abbauverhalten der Linker steuern.

Als Polypeptide werden vorzugsweise solche Moleküle eingesetzt, die zur Bildung definierter Homo- oder Hetero-Dimere neigen, insbesondere über Wasserstoffbrückenbindungen (wie z.B. zwischen Immunglobulin-Domänen) oder hydrophobe Wechselwirkungen (wie z.B. in den so genannten Leucin-Zippern). Auch hier werden gezielt solche Paare eingesetzt mit einem Schmelzpunkt im Bereich von 40-60°C besitzen, die also bei physiologischen überwiegend im gepaarten Zustand vorliegen, aber bei therapeutisch erzielbaren Temperaturen in ihre Monomere zerfallen. Dazu wird der eine Bindungspartner kovalent an die Nanopartikel gekoppelt, der andere kovalent mit einer therapeutisch wirksamen Substanz gekoppelt. Das Aufschmelzen der Bindung zwischen den beiden Peptidsträngen führt zu einer Entkopplung von Nanopartikeln und therapeutisch wirksamen Substanzen, die anschließend - ggf. erst nach beispielsweise enzymatischer Abspaltung - in frei diffundierbarer Form vorliegen.

Desgleichen können auch Polypeptid-Nukleinsäure-Wechselwirkungen in einem entsprechenden Linker genutzt werden. Dazu werden beispielsweise an die Nanopartikel Nukleinsäure-bindende Polypeptide gekoppelt, welche nicht-kovalent mit Nukleinsäuren wechselwirken. Diese Wechselwirkungen können ebenfalls durch Wärmeeinwirkung aufgeschmolzen werden, so dass die gebundene Nukleinsäure nebst angekoppeltem Effektormolekül freigesetzt wird. Gegebenenfalls kann auch die freigesetzte Nukleinsäure selbst als Effektormolekül wirken (beispielsweise siRNA, antisense DNA etc.). Als Nukleinsäure bindende Polypeptide kommen insbesondere Zink-Finger mit einer Länge zwischen 20 und 50 Aminosäuren in Betracht, aber auch das häufige Helix-Turn-Helix-Motiv DNA-bindender Domänen kommt in Frage oder das so genannte "single-strand binding protein" (SSB) zur DNA-Bindung (ein kleines Protein mit einer DNA-bindenden Domäne von ca. 100 Aminosäuren) bzw. das "RNA recognition motif" (RRM bzw. RNP-1) einzelstrang-RNA-bindender Proteine (in einem Umfang von ca. 90 Aminosäuren) oder das "double-stranded RNA binding motif" (DRBM) Doppelstrang-RNA-bindender Proteine (im Umfang von ca. 65 Aminosäuren).

Als weitere Möglichkeit kann schließlich auch die Bindung niedermolekularer Liganden durch Nukleinsäuren (Aptamere) bzw. Proteine in einem Linker-System genutzt werden. Grundsätzlich können alle Moleküle zum Einsatz kommen, indem beispielsweise Antikörper gegen ein solches so genanntes "Hapten" generiert werden (häufig eingesetzt werden beispielsweise Antikörper gegen Dinitrophenol, Trinitrophenol, Digoxigenin., Digoxin, Biotin). Praktikabel sind besonders auch Bindungstaschen von Biomolekülen, beispielsweise für Coenzyme (wie Coenzym A, ATP, GTP, FAD, NADH, NADPH, Biotin, Folsäure, Pyridoxalphosphat, etc.), Substrate (wie z.B. die 73 Aminosäuren umfassende Glutathion-Bindungsstelle der Glutathion-S-Transferase GST) oder Hormone (wie z.B. die Hormonbindungsdomäne der nukleären Hormonrezeptoren für Androgene, Östrogene, Retinsäure, Thyroxin, Vitamin D3 im Umfang von 218 bis 252 Aminosäuren). Eine der am häufigsten genutzten Wechselwirkungen und gleichzeitig die stärkste bekannte nicht-kovalente Bindung ist die des Biotins an das Avidin bzw. Streptavidin. Wegen der hohen Bindungsavidität ist hier möglicherweise auf modifiziertes Avidin bzw. auf Biotin-Analoga (etwa Desthiobiotin oder Iminobiotin) mit einer schwächeren Bindung auszuweichen, um ein Aufschmelzen im technisch erreichbaren Temperaturbereich zu erzielen. In allen Fällen ist es sinnvoll, den mikromolekularen Liganden an das Effektormolekül zu koppeln und den makromolekularen Liganden an die Nanopartikel; je nach Wahl des Liganden kann aber auch die umgekehrte Anordnung vorteilhaft sein.

Als Kopplungsmethoden kommen bei dieser bevorzugten Ausführungsform also nur solche in Frage, die eine Bindung zwischen Nanopartikel und Wirkstoff erzeugen, die unter "normalen" physiologischen Bedingungen eine ausreichende Stabilität aufweist, die aber unter den erfindungsgemäß angewandten Bedingungen (Impuls) wesentlich instabiler ist. Der eigentliche Mechanismus der Freisetzung und somit auch der Bindungstyp sind vom Zielort (z.B. Tumor bei Krebserkrankung) abhängig und muss durch die gängigen chemischen Kopplungsmethoden einstellbar sein. Ebenso kann die Freisetzung intrazellulär (z.B. in Tumorzellen) oder extrazellulär erfolgen. Die erfindungsgemäß hergestellten Teilchen unterscheiden sich also von bereits bekannten Wirkstoffträgern dadurch, dass erst durch Aktivierung im magnetischen Wechselfeld eine Wirksamkeit erreicht werden kann, während der Wirkstoff ohne diesen Impuls weitgehend inaktiv ist.

Erfindungsgemäß basieren die aktivierbaren Nanopartikel-Wirkstoff-Konjugate bevorzugt auf magnetischen eisenhaltigen Kernen, die von einer oder mehreren kolloidalen Hüllen oder Beschichtungen umgeben sind, welche eine Möglichkeit zur Ankopplung der Wirkstoffe über funktionelle Gruppen bieten. Der Kern besteht dabei vorzugsweise aus Magnetit oder Maghemit. Die primäre Funktion der Hüllen ist es, eine kolloidale Verteilung im wässrigen Medium zu erreichen und die Nanopartikel vor Agglomerationen zu schützen. Mehrschalig umhüllte Partikel, wie sie in WO 98/58673 beschrieben werden, sind prinzipiell als Basis für die aktivierbare Nanopartikel-Wirkstoff-Konjugate geeignet, da das biologische Verhalten solcher Partikel durch Überbeschichtungen mit Polymeren einstellbar und eine Ankopplung der Wirkstoffe an funktionelle Gruppen der Primärhülle möglich ist.

Die Wirkstoffe können durch unterschiedliche Methoden an die Primärhüllen angekoppelt werden. Im Falle einer Stabilisierung der Partikel-Kerne durch Bedingungen erfolgt. Ebenso ist es möglich, einen Wirkstoff an ein mit o.g. Gruppen funktionalisiertes Alkoxysilan zu koppeln (siehe Beispiel 1), wobei in einem nachfolgenden Schritt dieses Konjugat an die Schutzhülle von bereits durch Silane stabilisierten Partikeln gekoppelt wird. Dabei ist die Kopplung nicht auf kovalente Bindungen beschränkt. Erfindungsgemäß können auch ionische Wechselwirkungen ausreichender Stabilität erzeugt werden.

Eine weitere Beschichtung der aktivierbaren Nanopartikel-Wirkstoff-Konjugate (z.B. durch Polymere), wie sie in Patentschrift WO 98/58673 beschrieben wird, ist ebenfalls möglich, und kann genutzt werden, um die biologischen Eigenschaften der Partikel-Wirkstoff-Konjugate zu verbessern. Ebenso können weitere Moleküle angekoppelt werden, die dem Gesamtkonstrukt Zielfindungseigenschaften verleihen (z.B. polyklonale, Antikörper, monoklonale Antikörper, humanifizierte Antikörper, humana Antikörper, chimere Antikörper, rekombinante Antikörper, bispezifische Antikörper, Antikörperfragmente, Aptamere, Fab-Fragmente, Fc-Fragmente, Peptide, Peptidomimetika, gap-Mere, Ribozymes, CpG-Oligomere, DNA-Zyme, Riboswitches, oder Lipide). Voraussetzung ist, dass alle weiteren Modifizierungen die aktivierbare Freisetzung des Wirkstoffes am Zielort nicht behindern.

Als Linker können somit diverse Moleküle mit bis zu 500 Kohlenstoffatomen oder 10 bis 30 Basenpaaren, vorzugsweise 15 - 25 Basenpaaren oder 10 - 30 Aminosäuren, vorzugsweise 15 - 25 Aminosäuren dienen, vorausgesetzt, der Linker enthält eine thermisch, photochemisch oder enzymatisch spaltbare Gruppe, säurelabile Gruppe oder anders leicht ablösbare Gruppe. Eine Bindung im Linker-Molekül und/oder die Bindung des Linkers zum Wirkstoff und/oder die Bindung des Linkers zur Oberfläche des Nanopartikels müssen somit entweder durch die Einwirkung des magnetischen Wechselfeldes direkt oder indirekt spaltbar sein. Eine indirekte Spaltung ist dann gegeben, wenn beispielsweise durch das magnetische Wechselfeld Enzyme wie z.B. Peptidasen, Esterasen oder Hydrolasen am Zielort, z.B. in der Krebszelle angeregt oder deren Aktivität oder Expression gesteigert wird und diese Enzyme die vorgenannte Spaltung bewirken können. Zudem kann eine indirekte Spaltung bei der Verwendung von magnetischen Nanopartikeln erfolgen, wenn diese durch das magnetische Wechselfeld erwärmt werden und dadurch eine thermisch labile Bindung gespalten wird. Denkbar ist auch die Erhöhung des pH-Werte am Zielort durch Einwirkung des magnetischen Wechselfeldes und die nachfolgende Spaltung von säurelabilen Bindungen im Linker-Molekül.

Als enzymatisch spaltbare Gruppe im oder am Linker-Molekül sind die Estergruppe und die Amid- bzw. bzw. Peptidgruppe zu nennen. Thermisch oder mittels Säure spaltbare Gruppen umfassen z.B. Phosphatgruppen, Thiophosphatgruppen, Sulfatgruppen, Phosphamidgruppen, Carbamatgruppen oder Imingruppen.

Der Wirkstoff muss nicht notwendigerweise kovalent an den Linker gebunden werden, sondern kann auch ionisch oder über Wasserstoffbrücken gebunden oder interkaliert oder komplexiert vorliegen.

Ferner besteht auch die Möglichkeit, die Wirkstoffe adsorptiv an die Oberfläche der Nanopartikel zu binden und mit einer Barriereschicht zu überziehen, welche die Freisetzung des Wirkstoffs weitgehend verhindert bis die Barriereschicht durch Einwirkung eines magnetischen Wechselfeldes derart verändert insbesondere abgebaut wird, dass die Freisetzung des Wirkstoffs erfolgen kann.

In weiteren bevorzugten Ausführungsformen werden die erfindungsgemäßen Nanopartikel mit einer oder mehreren Hüllen oder Beschichtungen umgeben bzw. überzogen. Diese Hüllen oder Beschichtungen können eine oder mehrere Funktionen erfüllen und können als Schutzhülle, Barriereschicht oder zellselektive Beschichtung dienen.

Für den Fall einen nur schwachen Bindung der therapeutisch aktiven Substanzen an die Nanopartikel, beispielsweise bei einer nicht-kovalenten, ionischen, adsorptiven, lipophilen und/oder Van der Waalschen Bindung und/oder einen Anbindung über Wasserstoffbrücken kann eine Schutzhülle oder Barrierebeschichtung die Freisetzung der therapeutisch aktiven Substanzen unterbinden, bis die Nanopartikel ihren Bestimmungsort erreicht haben. Anstelle dieser Schutzhülle oder Barrierebeschichtung oder als weitere Schicht auf dieser Schutzhülle oder Barrierebeschichtung kann eine äußere Schicht aufgebracht werden, welche zellspezifische Funktionalitäten trägt.

Diese zellspezifische Beschichtung erhöht die Affinität der Nanopartikel zu bestimmten Zellen, beispielsweise zu bestimmten Bakterienzellen oder zu bestimmten Tumorzellen und dient somit der Zelldiskriminierung. Solche zellspezifischen Nanopartikel reichem sich bevorzugt in solchen Zellen an, zu denen sie aufgrund der Funktionalität auf ihrer Oberfläche eine erhöhte Affinität haben und sind somit tumorspezifisch. Mit dieser Technologie lassen sich beispielsweise tumorspezifische Nanopartikel für bestimmte Krebsarten entwickeln.

Ferner können auch die Nanopartikel durch eine kolloidale Schutzhülle stabilisiert werden, welche die Nanopartikel vor einer Agglomeration schützen. Es ist bevorzugt, wenn derartige Schutzhüllen oder Beschichtungen Aminogruppen oder Carboxygruppen aufweisen. Für die der Schutzhüllen bzw. Beschichtungen können biologische, synthetische oder semisynthetische Polymere eingesetzt werden. Für die Erzeugung einer Barriereschicht werden vorzugsweise biostabile, d.h. gegen biologischen Abbau weitgehend resistente Polymere eingesetzt. Zur Erzeugung von zellspezifischen Hüllen bzw. Beschichtungen werden vorzugsweise biologisch abbaubare Polymere verwendet.

Als biostabile Polymere können eingesetzt werden: Polyacrylsäure und Polyacrylate wie Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Polyethylen, Polypropylen, Polytetrafluorethylen, Polyurethane, Polyetherurethane Silicon-Polyetherurethane, Silicon-Polyurethane, Silicon-Polycarbonat-Urethane, Polyolefin-Elastomere, Polyisobutylene, EPDM-Gummis, Fluorosilicone, Carboxymethylchitosane, Polyaryletheretherketone, Polyetheretherketone, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Ethylvinylacetat-copolymere, Polysulfone, Epoxyharze, ABS-Harze, EPDM-Gummis, Silicone wie Polysiloxane, Polydimethylsiloxane, Polyvinylhalogene und Copolymere, Celluloseether, Cellulosetriacetate. Chitosane und Copolymere und/oder Mischungen dieser Substanzen.

Als bioabbaubare Polymere können verwendet werden: Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride wie Polymaleinsäureanhydride, Polyhydroxymethacrylate, Fibrin, Polycyanoacrylate, Polycaprolactondimethylacrylate, Poly-ß-Maleinsäure Polycaprolactonbutylacrylate, Multiblockpolymere wie z.B. aus Oligocaprolactondiole und Oligodioxanondiole, Polyetherestermultiblockpolymere wie z.B. PEG und Poly(butylenterephtalat), Polypivotolactone, Polyglycolsäuretrimethylcarbonate Polycaprolacton-glycolide, Poly(γ-ethylglutamat), Poly(DTH-iminocarbonat), Poly(DTE-co-DT-carbonat), Poly(bisphenol A-iminocarbonat), Polyorthoester, Polyglycolsäuretrimethylcarbonate, Polytrimethylcarbonate Polyiminocarbonate, Poly(N-vinyl)-pyrrolidon, Polyvinylalkohole, Polyesteramide, glycolierte Polyester, Polyphosphoester, Polyphosphazene, Poly[(p-carboxyphenoxy)propan] Polyhydroxypentansäure, Polyanhydride, Polyethylenoxidpropylenoxid, weiche Polyurethane, Polyurethane mit Aminosäurereste im Backbone, Polyetherester wie das Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, Hyaluronsäure, Chitosan und seine Derivate, Heparansulfate und seine Derivate, Heparine, Chondroitinsulfat, Dextran, ß-Cyclodextrine, Alginate, Glycosaminoglycane, Saccharide, Polysaccharide, Proteoglykane, Glycoproteine, Copolymere mit PEG und Polypropylenglycol, Gummi arabicum, Guar, Gelatine, Collagen Collagen-N-Hydroxysuccinimid, Lipide, Phospholipide, Modifikationen und Copolymere und/oder Mischungen der vorgenannten Substanzen.

Zur weiteren Affinitätssteigerung bezüglich bestimmter Zellen können auf der Oberfläche der Nanopartikel bzw. auf der äußeren Schicht oder Hülle der Nanopartikel monoklonale Antikörper und/oder Aptamere gekoppelt werden. Die monoklonalen Antikörper und Aptamere sind derart ausgestaltet, dass sie bestimmte Zellen beispielsweise Tumorzellen erkennen und die Zelidiskriminierung der Nanopartikel weiter steigern.

In einer bevorzugten Ausführungsform der vorliegenden Erfindung bestehen die Kerne der magnetischen Nanopartikel aus Magnetit (Fe₃O₄). Maghemit (γ-Fe₂O₃) oder Mischungen dieser beiden Oxide und sind vorzugsweise superparamagnetisch. Die Kerne sind darüber hinaus durch kolloidale Schutzhüllen stabilisiert, die eine Anbindung der therapeutisch wirksamen Substanzen ermöglichen. Die Konjugate aus magnetischen Nanopartikeln und therapeutisch wirksamen Substanzen sind durch die Art der Bindung so konstruiert, dass eine gezielte Freisetzung der therapeutisch wirksamen Substanz im menschlichen Körper mittels eines externen Magnetwechselfeldes (Impuls) möglich ist.

Des Weiteren betrifft die vorliegende Erfindung pharmazeutische Zusammensetzungen, welche die erfindungsgemäßen Nanopartikel enthalten sowie die Verwendung der erfindungsgemäßen Nanopartikel zur Herstellung derartiger pharmazeutischer Zusammensetzungen.

Bei diesen pharmazeutischen Zusammensetzungen handelt es sich insbesondere um Infusions- oder Injektionslösungen. Derartige Lösungen der Nanopartikel in beispielsweise physiologischer Kochsalzlösung sind für die interstitielle bzw. intratumorale Applikation geeignet. Eine intraarterielle oder intravenöse Applikation ermöglicht ferner eine systemische, den ganzen Körper betreffende Therapiemöglichkeit für nicht solide und/oder metastasenbildende Tumorarten.

Die erfindungsgemäßen Nanopartikel und pharmazeutischen Zusammensetzungen werden für die Behandlung als auch zur Prophylaxe von Krankheiten eingesetzt, welche sich durch entartete Zellspezies oder körperfremde Zellen auszeichnen und bei denen die Eigenschaften der erfindungsgemäßen Nanopartikel ausgenutzt werden können, zwischen fremden Zellen bzw. entarteten Zellen und gesunden körpereigenen Zellen zu diskriminieren. Als entartete Zellen gelten insbesondere Krebszellen bzw. in ihrer Proliferation gestörte Zellen als auch stenotisches oder restenotisches Gewebe. Als körperfremde Zellen könne insbesondere Bakterien genannt werden.

Demzufolge werden die erfindungsgemäßen Nanopartikel und die Nanopartikel enthaltenden pharmazeutischen Zusammensetzungen zur Prophylaxe und Behandlung von Tumoren, Karzinomen und Krebs eingesetzt.

Als Beispiele für Krebs- und Tumorarten, wo die erfindungsgemäßen Nanopartikel eingesetzt werden können, sind zu nennen: Adenokarzinome, Aderhautmelanom, Akute Leukämie, Akustikusneurinom, Ampullenkarzinom, Analkarzinom, Astrozytome, Basaliom, Bauchspeicheldrüsenkrebs, Bindegewebstumor, Blasenkrebs, Bronchialkarzinom, Nicht-kleinzelliges Bronchialkarzinom, Brustkrebs, Burkitt-Lymphom, Corpuskarzinom, CUP-Syndrom, Dickdarmkrebs, Dünndarmkrebs, Dünndarmtumore, Eierstockkrebs, Endometriumkarzinom, Ependymom, Epithel-Krebsarten, Ewing-Tumoren, Gastrointestinale Tumoren, Gallenblasenkrebs, Gallenkarzinome, Gebärmutterkrebs, Gebärmutterhalskrebs, Glioblastome, Gynäkologische Tumoren, Hals-, Nasen- und Ohrentumoren, Hämatologische Neoplasien, Haarzell-Leukämie, Harnröhrenkrebs, Hautkrebs, Himtumoren (Gliome), Himmetastasen, Hodenkrebs, Hypophysentumor, Karzinoide, Kaposi-Sarkom, Kehlkopfkrebs, Keimzellentumor, Knochenkrebs, kolorektales Karzinom, Kopf-Hals-Tumore (Tumore des Hals- Nasen- und Ohrenbereichs), Kolonkarzinom, Kraniopharyngeome, Krebs im Mundbereich und auf der Lippe, Leberkrebs, Lebermetastasen, Leukämie, Lidtumor, Lungenkrebs, Lymphdrüsenkrebs (Hodgkin/Non-Hodgkin), Lymphome, Magenkrebs, Malignes Melanom, malignes Neoplasma, Malignome des Magen-Darm-Traktes, Mammakarzinom, Mastdarmkrebs, Medulloblastome, Melanom, Meningeome, Morbus Hodgkin, Mycosis fungoides, Nasenkrebs, Neurinom, Neuroblastom, Nierenkrebs, Nierenzellkarzinome, Non-Hodgkin-Lymphome, Oligodendrogliom, Ösophaguskarzinom, osteolytische Karzinome. u. osteoplastische Karzinome, Osteosarkom, Ovarial-Karzinom, Pankreaskarzinom, Peniskrebs, Plasmozytom, Plattenepithelkarzinome des Kopfes und Halses, Prostatakrebs, Rachenkrebs, Rektumkarzinom, Retinoblastom, Scheidenkrebs, Schilddrüsenkarzinom, Schneeberger Krankheit, Speiseröhrenkrebs, Spinaliom, T-Zell-Lymphom (Mycosis fungoides), Thymom, Tubenkarzinom, Tumoren des Auges, Urethrakrebs, Urologische Tumoren, Urothelkarzinom, Vulvakrebs, Warzenbeteiligung, Weichteiltumoren, Weichteilsarkom, Wilms Tumor, Zervixkarzinom und Zungenkrebs.

Bevorzugt sind insbesondere solide Tumoren. Ferner sind bevorzugt Prostatakarzinome, Gehirntumore, Sarkome, Zervix-Karzinome, Ovarialkarzinome, Mammakarzinome, Bronchialkarzinome, Melanome, Kopf-Hals Tumore, Ösophaguskarzinome, Rektumkarzinome, Pankreas-, Blasen- und Nierenkarzinome, Metastasen der Leber, des Gehirns und in Lymphknoten.

Insbesondere bevorzugt ist ferner die Anwendung und der Einsatz der erfindungsgemäßen Nanopartikel zusammen mit der konventionellen Hyperthermie, Strahlentherapie und/oder zusammen mit der herkömmlichen Chemotherapie.

### Beispiele

### Beispiel 1:

### Herstellung von Nanopartikeln mit angekoppeltem Mitomycin zur Freisetzung:

Zur Ankopplung des Zytostatikums Mitomycin an Aminosilan-stabilisierte Eisenoxid-Nanopartikel wird zunächst ein Konjugat aus Mitomycin und Triethoxysilylbutyraldehyd synthetisiert. Dazu werden Mitomycin und Triethoxysilylbutyraldehyd im molaren Verhältnis von 1:1 gelöst und für 2 Stunden gerührt. Auf diese Weise wird der Wirkstoff über eine Imin-Bindung an das Silan gekoppelt. Dieses Konjugat wird dann folgendermaßen für die Beschichtung von Eisenoxid-Nanopartikeln eingesetzt: Eine Suspension unbeschichteter Eisenoxid-Nanopartikel (hergestellt aus Eisen(II)Chlorid und Eisen(III)Chlorid durch Fällung mit Natriumhydroxid) wird mit Essigsäure auf einen pH-Wert von 5 eingestellt. Dann wird unter ständigem Rühren eine Mischung aus dem Mitomycin-Silan-Konjugat und Aminopropyltriethoxysilan zugegeben. Das molare Verhältnis von Mitomycin zu Aminopropyltriethoxysilan wird vorher auf 1:50 eingestellt. Nach 24 Stunden wird Ethylenglykol zugegeben, so dass sich das Volumen der Suspension verdoppelt. Danach wird das Wasser destillativ entfernt. Dadurch werden die Silane fest an die Eisenoxid-Partikel gekoppelt. Die Suspension wird durch Dialyse gegen Reinstwasser gereinigt und auf eine Eisenkonzentration von 1 mol/l aufkonzentriert (destillativ).

### Beispiel 2:

### Ankopplung eines Amino-modifizierten Oligonukleotids an Eisenoxid-Nanopartikel über Glutaraldehyd als Linker

Aminosilan-stabilisierte Nanopartikel werden durch Fällung von Eisen(II)Chlorid und Eisen(III)Chlorid mit Natriumhydroxid hergesellt und durch Zugabe von Aminpropyltriethoxysilan beschichtet (gemäß WO 97/38058). Die Suspension wird auf eine Eisenkonzentration von 2 mol/l aufkonzentriert.

500µl der Suspension werden mit 10ml PIPES-Puffer (Piperazin-N,N'-bis-2-ethansulfonsäure; pH = 7,4) gewaschen. Dann wird 5-prozentige GlutaraldehydLösung (6ml) zugegeben und für 2 Stunden gerührt. Die so aktivierten Partikel werden gewaschen und in 800µl PIPES-Puffer resuspendiert. 0,3 µmol des Amino-modifizierten Oligonukleotids (Amino-Endmodifizierung) werden in Wasser gelöst und zugegeben. Die Suspension wird für 12 Stunden gerührt. Danach werden die Partikel mit Reinstwasser gewaschen und in 500µl Reinstwasser resuspendiert.

### Beispiel 3:

### Aufbringung einer biologisch abbaubaren Schicht

Die Nanopartikel mit Glutardialdehyd-Linkem und daran immobilisiertem Oligonukleotid hergestellt gemäß Beispiel 2 werden gefriergetrocknet und im Sprühverfahren mit einer ethanolischen Lösung enthaltend Polyglycol behandelt. Nach der Entfernung des Lösungsmittels werden mit einer biologisch abbaubaren Polyglycolbeschichtung versehene Nanopartikel erhalten. Derartige Beschichtungen dienen zur Anbindung von beispielsweise Aptameren und tumorzellspezifischen Antikörpern.

### Beispiel 4:

### Kopplung von Wirksubstanzen über Oligonukleotide

Die Synthese von Oligonukleotiden erfolgt heute weitestgehend automatisiert unter Anwendung einer etablierten Schutzgruppenchemie. Ein kurzes Oligonukleotid bestehend aus 15 Nukleotiden wird über eine Endmodifizierung kovalent an die Nanopartikel gekoppelt (siehe Beispiel 2). Ein zweites, zum ersten komplementäres Oligonukleotid ist über eine Endmodifizierung mit dem Wirkstoff Doxorubicin gekoppelt. Beide Komponenten werden zusammengebracht und kurz auf 95°C erhitzt, um die Oligonukleotide zu denaturieren. Durch anschließende Inkubation bei einer Temperatur knapp unter dem Schmelzpunkt des Oligonukleotids paaren sich beide Stränge zu einem Doppelstrang. Die Sequenz der Oligonukleotide ist dabei so ausgewählt, dass sich unter physiologischen Bedingungen ein Schmelzpunkt von ca. 48°C ergibt, also kein Aufschmelzen des Doppelstranges möglich ist. Durch eine Erwärmung auf über 50°C wird der entstandene DNA-Doppelstrang quantitatitv aufgeschmolzen und der Wirkstoff wird zusammen mit dem anhängenden Oligonukleotid freigesetzt. Die einzelsträngige DNA wird, wenn sie ins Innere einer Zelle gelangt, schnell abgebaut, so dass dann der Wirkstoff gänzlich frei ist.

### Beispiel 5:

### Kopplung von Wirksubstanzen über Nukleinsäure-Tripelhelices

Doppelsträngige RNA kann als so genannte siRNA (für small interfering RNA) therapeutisch eingesetzt werden, um spezifisch Gene abzuschalten. Soll eine solche RNA von dem als Transporter eingesetzten Nanopartikel extern gesteuert freigesetzt werden, ist die Bindung über eine spezifische Tripel-Helix die Methode der Wahl. Ein zur eingesetzten siRNA passendes Doppelstrang-bindendes Oligonukleotid wird über eine Endmodifizierung des Oligonukleotids kovalent an die Nanopartikel gebunden (gemäß Beispiel 2). (Dadurch wird die spätere Bildung eines so genannten "triplet forming oligonucleotide" (TFO) ermöglicht). Um eine erhöhte Stabilität gegenüber hydrolytischen Enzymen zu erzielen, werden dabei Oligonukleotide eingesetzt, bei denen das Zucker-Phosphat-Rückgrat der Nukleinsäuren durch ein synthetisches peptidartiges Rückgrat ersetzt ist, das den Nukleinsäuren strukturanalog ist; es handelt sich hierbei um so genannte Peptid-Nukleinsäuren (PNAs). Durch Hybridisierung knapp unter dem Schmelzpunkt der angestrebten Tripelhelix, (der gleichzeitig niedriger ist als der Schmelzpunkt der doppelsträngigen RNA), wird das kovalent gebundene Oligonukleotid die doppelsträngige RNA in der breiten Furche binden.

Weil der Schmelzpunkt von hier 45°C nicht erreicht wird, findet unter physiologischen Bedingungen keine nennenswerte Freisetzung statt. Erst durch die therapeutische Überschreitung des Schmelzpunktes der Tripelhelix schmilzt diese unter Freisetzung der doppelsträngigen siRNA auf.

### Beispiel 6:

### Kopplung von Wirksubstanzen über ein Oligopeptid-Molekül

Besonders zum Targeting von gentechnisch hergestellten Polypeptid-Effektoren wie etwa dem Tumor-Nekrose-Faktor (TNFalpha) eignet sich die temperatursensitive Kopplung über eine temperatursensitive Oligopeptid-Domäne. Hierbei wird ein heterodimerisierender so genannter Leucin-Zipper eingesetzt. Die Bindung wird stabilisiert und gleichzeitig spezifiziert durch die ionischen Wechselwirkungen geladener Gruppen (Arginin/Lysin versus Glutamat/Aspartat).

An den Nanopartikeln wird ein synthetisches Oligopeptid aus 22 Aminosäuren des max-Leucin-Zippers über eine terminale Modifikation des Oligopeptids kovalent gebunden. Bei Zugabe einer gentechnisch hergestellten TNF-Präparation, die terminal die entsprechenden 22 Aminosäuren des myc-Leucin-Zippers trägt, wird der Tumornekrosefaktor quantitativ an die Nanopartikel gebunden. Während einer Thermotherapie wird die Schmelztemperatur der Leucin-Zipper überschritten, so dass der (in seiner Funktion nicht beeinträchtigte) Tumornekrosefaktor lokal freigesetzt wird.

### Beispiel 7:

### Kopplung von Wirksubstanzen über Oligonukleotid-Peptid-Brücken

Neben den spezifischen thermolabilen Wechselwirkungen von Nukleinsäuren mit Nukleinsäuren und von Proteinen mit Proteinen (bzw. Polypeptiden mit Polypeptiden) gibt es ebenso spezifische (wie auch unspezifische) biologische Wechselwirkungen von Proteinen bzw. Polypeptiden mit Nukleinsäuren. Da solche Wechselwirkungen genau auf den selben nicht-kovalenten Bindungen beruhen, sind sie grundsätzlich genau so thermolabil, wie die vorgenannten und lassen sich daher genau so als thermolabiles Linkersystem zur thermischen Freisetzung von Wirkstoffen nutzen. Es werden Proteine eingesetzt, die entweder unspezifisch (z.B. Histone, oder das einzelstrangbindende SSB-Protein der DNA-Replikationsgabel) oder hochspezifisch mit Nukleinsäuren wechselwirken (z.B. Repressoren, Transkriptionsfaktoren). Als spezifische DNA-bindende Polypeptide werden auch die so genannten "Helix-Turn-Helix"-Motive von Repressor-Proteinen sowie die so genannten "Zink-Finger"-Motive der nukleären Rezeptorproteine eingesetzt. Beide umfassen typischerweise um 60 Aminosäuren. (Zink-Finger-Motive bestehen aus zwei gleich großen Schleifen mit je zwei Paaren von Cysteinen, bzw. einem Paar Cysteine und einem Paar Histidine, welche über je ein komplexiertes Zink-Atom zusammengehalten werden). Dadurch entstehen zwei fingerartige Strukturen, die in die großen Furchen der DNA greifen. Zwischen den beiden Strukturen liegt ein 15 bis 20 Aminosäuren umfassender Linker, welcher bei den Steroidhormonrezeptoren eine Aminosäuresequenz enthält, die spezifisch eine palindromische DNA-Sequenz erkennt.

An die Oberfläche der Nanopartikel wird ein synthetisches Oligopeptid aus 60 Aminosäuren kovalent angekoppelt, welches das vollständige Zink-Finger-Motiv des Glucocorticoid-Rezeptors umfasst. Das Wirkstoffmolekül Doxorubicin wird kovalent an ein doppelsträngiges Oligonukleotid aus 15 Basenpaaren angekoppelt, welches die Erkennungssequenz des Glucocorticoid-Rezeptors umfasst (das so genannte "glucocorticoid response element" GRE). Beide Komponenten werden zu einem Komplex gekoppelt der unter physiologischen Bedingungen stabil ist. Werden die Nanopartikel durch Einkoppeln eines magnetischen Wechselfeldes erwärmt, wird die Schmelztemperatur des Komplexes überschritten. Durch den Zerfall des Komplexes wird das Oligonukleotid-Wirkstoff-Konjugat freigesetzt.

### Beispiel 8: Kopplung von Wirksubstanzen über Hapten-Antikörper-Brücken

Die spontane Bindung eines Haptens als Therapeutikum an körpereigene Eiweiße kann zu einer Immunreaktion führen. Die Anbindung von Antikörpern kann auch zu einer Neutralisierung des Wirkeffektes führen. Dieser Effekt wird genutzt, um durch eine thermische Zersetzung von Hapten-Antikörer-Komplexen eine lokale Aktivierung zu erreichen.

Biochemisch (oder wahlweise gentechnisch) erzeugte so genannte Fv-Fragmente (die kleinstmöglichen antigenbindenden Spaltstücke von Antikörpern) eines gegen Doxorubicin gerichteten Antikörpers werden kovalent an die Oberfläche von Nanopartikeln gebunden. Durch Zugabe eines Überschusses an Doxorubicin werden die Antigenbindungsstellen gesättigt. Durch magnetische Separation oder Zentrifugation werden die Doxorubicin-gesättigten Nanopartikel von unspezifisch gebundenem Wirkstoff befreit und ggf. zusätzlich gewaschen.

Nach intravenöser Gabe der floxorubicin-gesättigten Nanopartikel zirkulieren diese weitestgehend frei von den üblichen Nebenwirkungen des Zytostatikums. Es wird eine unspezifische Anreicherung der Nanopartikel im Bereich von Tumoren erreicht, weil die Nanopartikel dort durch die ständig neugebildeten, noch durchlässigen Gefäßwände die Gefäße verlassen können. Zusätzlich kann durch eine spezielle Oberflächenbeschichtung die intrazelluläre Aufnahme in Tumorzellen (bedingt durch die Häufigkeit der Mitose), aber nicht in gutartigen Zellen, bewirkt werden. Nach einer angemessenen Zeit der intratumoralen Anreicherung kann über externe Magnetfelder eine Erwärmung der Nanopartikel bewirkt werden; neben der dadurch erreichbaren Gewebeschädigung durch Hyperthermie wird der Hapten-Antikörper(-fragment)-Komplex durch die Erwärmung aufgeschmolzen. Auf Grund seiner autonomen zytotoxischen sowie der strahlungssensitivierenden Wirkung des Doxorubicins wird die gewebeschädigende Wirkung der Hyperthermie potenziert. Es wird somit eine echte Synergie der Tumorbekämpfung erzielt.

### Beispiel 9: Kopplung von Wirksubstanzen über Biotin-Avidin-Brücken

Die nicht-kovalente Bindung zwischen dem Vitamin Biotin und dem Bindungsprotein Avidin aus Hühner-Eiklar (bzw. seinem bakteriellen Analogon Streptavidin) ist die stärkste bekannte nicht-kovalente Wechselwirkung überhaupt. Wegen der hohen Bindungsenergie ist diese Bindung allerdings nicht im zur Verfügung stehenden Temperaturintervall aufschmelzbar. Um dennoch diese hochspezifische Bindung nutzen zu können, muss auf Derivate des Biotins mit verringerter Bindungsstärke zurückgegriffen werden, etwa das Desthiobiotin (mit einer Dissoziationskonstante von 5 × 10¹³ gegenüber 1 × 10¹⁵ beim Biotin) oder das Iminobiotin (Dissoziationskonstante 3,5 × 10¹¹), deren Bindung an (Strept-)Avidin bei therapeutisch erreichbaren Temperaturen physiologisch aufschmilzt.

Iminobiotin wird über seine ε-Aminogruppe mit der Aminogruppe des Doxorubicins gekoppelt; die Bindung wird dabei über Glutardialdehyd (hergestellt. Die Kopplung der Nanopartikel mit handelsüblichem Streptavidin erfolgt über eine Aminofunktion der Oberflächenbeschichtung ebenfalls mittels Glutardialdehyd. Durch Zugabe eines Überschusses an Iminobiotinyl-Doxorubicin werden die Nanopartikel mit Doxorubicin beladen. Diese Doxorubicin-beladenen Nanopartikel werden in vivo auf Grund der Durchlässigkeit der Endothelien im Tumorbereich passiv angereichert und zusätzlich durch Endozytose in den Tumorzellen auch aktiv angereichert. Die magnetisch induzierte Hyperthermie wird auch hier durch die thermische Freisetzung des Sensitizers Doxorubicin synergistisch verstärkt.

### Beispiel 10:

### Herstellung von Nanopartikeln mit angekoppeltem Cisplatin zur Freisetzung:

Zur Ankopplung des Zytostatikums Cisplatin an Aminosilan-stabilisierte Eisenoxid-Nanopartikel werden die in Beispiel 1 bezeichneten Nanopartikel zuerst mittels Aminopropyltriethoxysilan derivatisiert. Dazu wird eine Suspension unbeschichteter Eisenoxid-Nanopartikel (hergestellt aus Eisen(II)Chlorid und Eisen(III)Chlorid durch Fällung mit Natriumhydroxid) wird mit Essigsäure auf einen pH-Wert von 5 eingestellt. Aminopropyltriethoxysilan wird im molaren Verhältnis bezogen auf die theoretisch maximale Anzahl an Hydroxygruppen tropfenweise zugesetzt, eine Stunde bei Raumtemperatur gerührt und anschließend mit einer equimolaren Menge an Cisplatin versetzt, welche eine nukleophile Substitutionsreaktion mit der Aminogruppe des Silans eingeht.

Die erhaltenen derivatisierten Nanopartikel haben folgende Struktur:

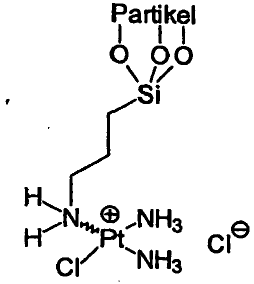

### Beispiel 11: Wirkung der Cisplatin-Nanopartikel gemäß Beispiel 10 auf Glioblastom-Zellen

Eine wäßrige Lösung dieser Cisplatin-Nanopartikel wurde im Vergleich zu nicht derivatisierten Nanopartikeln bei Glioblastom-Zellen untersucht.

Die in-vitro Untersuchungen wurden mit der Glioblastom-Human-Zelllinie RUSIRS1 (Hirntumor) durchgeführt. Die Glioblastomzellen wurden wie in DE 199 12 798 C1 beschrieben aus dem Tumorgewebe eines Patienten entnommen und kultiviert. Zur Testung der Wirksamkeit der Cisplatin-Nanopartikel wurden jeweils 2×10⁶ RUSIRS1 Zellen in einer 75 cm³ Zellkulturflasche mit 25 ml Zellkulturmedium (D-MEM + 20% FBS + 1,2 ml Pyruvat) angesetzt. Die Zellsuspension wurde gleichmäßig auf 4 Kulturgefäße verteilt. Zu zweien dieser Zellsuspensionen wurden jeweils 153 µl wäßrige Lösung dieser Cisplatin-Nanopartikel (c_{Fe} = 2 mol/l) gegeben. Die anderen beiden Kulturflaschen dienten als Referenz und es wurden dort 153 µl wäßrige Lösung nicht derivatisierter Nanopartikel (c_{Fe} = 2 mol/l) zugesetzt. Die Proben der Nanopartikel wurden vor der Zugabe zu den Zellen für 15 Minuten auf 37°C erwärmt und 10 Minuten bei RT stehen gelassen. Nach der Zugabe der Nanopartikel wurden die Proben 1 h stehen gelassen und danach einer 30 minütigen Behandlung durch ein magnetisches Wechselfeld ausgesetzt. Diese Behandlung wurde nach 24 Stunden wiederholt. Nach bereits 48 Stunden Inkubationszeit bei 37°C zeigten sich bei den beiden Proben mit Cisplatin-Nanopartikeln bereits deutlich ausgeprägtere Schädigungen als bei den beiden Proben mit nicht derivatisierten Nanopartikeln.

## Patentansprüche

1. Nanopartikel, wobei an das Nanopartikel mindestens eine therapeutisch wirksame Substanz über ein Linker-Molekül gebunden ist, wobei das Linker-Molekül eine thermolabile, elektro-magneto-Iabile, photolabile, säurelabile, interkalierbare oder enzymatisch spaltbare Gruppe enthält und wobei das Linker-Molekül ein Nukleinsäuremolekül, ein Polypeptid, eine Peptid-Nukleinsäure, ein Aptamer, DNA, RNA, ein Leucin-Zipper, ein Oligonukleotid, ein Oligopeptid, eine Hapten-Antikörper-Brücke oder eine Biotin-Avidin-Brücke ist, oder wobei im Linker-Molekül eine Bindung von Biotin an Avidin oder Streptavidin vorliegt, und wobei die Ablösung der mindestens einen therapeutisch wirksamen Substanz von dem Nanopartikel durch ein magnetisches Wechselfeld bewirkt oder initiiert oder wesentlich gesteigert wird.

2. Nanopartikel nach Anspruch 1, wobei die mindestens eine therapeutisch wirksame Substanz kovalent an den Nanopartikel gebunden ist.

3. Nanopartikel nach einem der vorherigen Ansprüche, wobei das Nukleinsäuremolekül ein doppelsträngiges Nukleinsäure-Konstrukt, eine Doppelhelix, ein Homo-oder Hetero-Hybrid aus DNA-DNA, DNA-RNA, DNA-PNA, RNA-RNA, RNA-PNA oder PNA-PNA ist.

4. Nanopartikel nach einem der vorherigen Ansprüche, wobei der Nanopartikel mit einer Schutzhülle oder einer Beschichtung versehen ist.

5. Nanopartikel nach Anspruch 4, wobei die Schutzhülle oder Beschichtung Aminogruppen oder Carboxygruppen aufweist.

6. Nanopartikel nach einem der vorherigen Ansprüche, wobei die mindestens eine therapeutisch wirksame Substanz ausgewählt wird aus der Gruppe umfassend anti-proliferative, anti-migrative, anti-angiogene, anti-thrombotische, antiinflammatorische, antiphlogistische, zytostatische, zytotoxische, anti-koagulative, anti-bakterielle, anti-virale und/oder anti-mykotische Wirkstoffe.

7. Nanopartikel nach Anspruch 6, wobei die mindestens eine therapeutisch wirksame Substanz ausgewählt wird aus der Gruppe umfassend Actinomycin D, Ametantron, 9-Aminocamptothecin, Aminoglutethimid, Amsacrin, Anastrozol, Antagonisten von Purin- und Pyrimidin-Basen, Anthracycline, Aromatasehemmer, Asparaginase, Antiöstrogene, Bendamustin, Bexaroten, Biolimus A9, Bleomycin, Buselerin, Busulfan, Calicheamicine, Camptothecin, Camptothecin-Derivate, Capecitabin, Carboplatin, Carmustin, Chlorambucil, Cisplatin, Cladribin, Cyclophosphamid, Cytarabin, Cytosinarabinosid, alkylierende Cytostatika, Dacarbazin, Dactinomycin, Daunorubicin, 5'-Deoxy-5fluorouridin, Docetaxel, Doxorubicin (Adriamycin), Doxorubicin lipo, Epirubicin, Estramustin, Etoposid, Exemestan, Fludarabin, Fluorouracil, Folsäureantagonisten, Formestan, Gemcitabin, Glucocorticoide, Goselerin, Hormone und Hormonantagonisten, Hycamtin, Hydroxyharnstoff, Idarubicin, Ifosfamid, Imatinib, Irinotecan, Letrozol, Leuprorelin, Lomustin, Maytansinoide, Melphalan, Mercaptopurin, Methotrexat, Miltefosin, Mitomycine, Mitopodozid, Mitosehemmstoffe, Mitoxantron, Nimustin, Oxaliplatin, Oxazaphosphorine, Paclitaxel, Pentostatin, Podophyllotoxin-Derivate, Procarbazin, Rapamycin, Rhodomycin D, Tamoxifen, Temozolomid, Teniposid, Testolacton, Thiotepa, Thioguanin, Topoisomerase-Inhibitoren, Topotecan, Treosulfan, Tretinoin, Triptorelin, Trofosfamide, Vinca-Alkaloide, Vinblastin, Vincristin, Vindesin, Vinorelbin, zytostatisch wirksame Antibiotika.

8. Nanopartikel nach Anspruch 6, wobei die mindestens eine therapeutisch wirksame Substanz ausgewählt wird aus der Gruppe umfassend Nukleinsäuren, Aminosäuren, Peptide, Proteine, Kohlenhydrate, Lipide, Glycoproteine, Glycane oder Lipoproteine, wobei die vorgenannten Stoffe anti-proliferative, anti-migrative, anti-angiogene, anti-thrombotische, antiinflammatorische, antiphlogistische, zytostatische, zytotoxische, anti-koagulative, anti-bakterielle, anti-virale und/oder anti-mykotische Eigenschaften besitzen.

9. Nanopartikel nach einem der vorherigen Ansprüche, wobei der Nanopartikel aus superparamagnetischen Eisenoxiden oder aus reinem Eisen mit einer Oxidschicht besteht.

10. Nanopartikel nach einem der vorherigen Ansprüche, wobei an den Nanopartikel ein Sensitizer, Radiosensitizer und/oder Verstärker zur Unterstützung der konventionellen Krebsbehandlungsmethoden gebunden ist.

11. Nanopartikel nach einem der vorherigen Ansprüche, wobei an die Oberfläche des Nanopartikels monoklonale Antikörper bzw. Antikörperfragmente und/oder Aptamere gekoppelt sind.

12. Infusionslösung enthaltend Nanopartikel gemäß eines der Patentansprüche 1 - 11.

13. Verwendung der Nanopartikel gemäß eines der Patentansprüche 1 - 11 zur Herstellung einer pharmazeutischen Zusammensetzung zur Behandlung und/oder Prophylaxe von proliferativen Erkrankungen, Krebs und bakteriellen Infektionen.

## Claims

1. Nanoparticle, wherein at least one therapeutically active substance is bound to the nanoparticle via a linker molecule, wherein the linker molecule contains a group which is thermolabile, electromagnetically labile, photolabile, acid-labile, or may be intercalated or enzymatically cleavable, and wherein the linker molecule is a nucleic acid molecule, a polypeptide, a peptide nucleic acid, an aptamer, DNA, RNA, a leucine zipper, an oligonucleotide, an oligopeptide, a haptene antibody bridge or a biotin avidin bridge, or wherein the linker molecule contains a bond of biotin to avidin or streptavidin, and wherein the separation of the at least one therapeutically active substance from the nanoparticle is caused or initiated or substantially enhanced by an alternating magnetic field.

2. Nanoparticle according to claim 1, wherein the at least one therapeutically active substance is covalently bound to the nanoparticle.

3. Nanoparticle according to any one of the preceding claims, wherein the nucleic acid molecule is a double stranded nucleic acid construct, a double helix, a homo- or a hetero-hybrid from DNA-DNA, DNA-RNA, DNA-PNA, RNA-RNA, RNA-PNA or PNA-PNA.

4. Nanoparticle according to any one of the preceding claims, wherein the nanoparticle is provided with a protective sheath or with a coating.

5. Nanoparticle according to claim 4, wherein the protective sheath or coating has amino groups or carboxy groups.

6. Nanoparticle according to any one of the preceding claims, wherein the at least one therapeutically active substance is selected from the group comprising antiproliferative, antimigration, antiangiogenic, antithrombotic, anti-inflammatory, antiphlogistic, cytostatic, cytotoxic, anticoagulative, antibacterial, antiviral and/or antimycotic agents.

7. Nanoparticle according to claim 6, wherein the at least one therapeutically active substance is selected from the group comprising actinomycin D, ametantrone, 9-Aminocamptothecin, aminoglutethimide, amsacrine, anastrozole, antagonists of purine and pyrimidine bases, anthracycline, aromatase inhibitors, asparaginase, antiestrogens, bendamustine, bexarotene, biolimus A9, bleomycin, buserelin, busulfan, calicheamicins, camptothecin, camptothecin derivatives, capecitabine, carboplatin, carmustine, chlorambucil, cisplatin, cladribine, cyclophosphamide, cytarabine, cytosine arabinoside, alkylating cytostatics, dacarbazine, dactinomycin, daunorubicin, 5'-deoxy-5-fluorouridine, docetaxel, doxorubicin (adriamycin), doxorubicin lipo, epirubicin, estramustine, etoposide, exemestane, fludarabine, fluorouracil, folic acid antagonists, formestane, gemcitabine, glucocorticoids, goserelin, hormones and hormone antagonists, hycamtin, hydroxyurea, idarubicin, ifosfamide, imatinib, irinotecan, letrozole, leuprorelin, lomustine, maytansinoids, melphalan, mercaptopurine, methotrexate, miltefosine, mitomycins, mitopodozide, antimitotic agents, mitoxantrone, nimustine, oxaliplatin, oxazaphosphorines, paclitaxel, pentostatin, podophyllotoxin derivatives, procarbazine, rapamycin, rhodomycin D, tamoxifen, temozolomide, teniposide, testolactone, thiotepa, thioguanine, topoisomerase inhibitors, topotecan, treosulfan, tretinoin, triptorelin, trofosfamides, vinca alkaloids, vinblastine, vincristine, vindesine, vinorelbine, cytostatically active antibiotics.

8. Nanoparticle according to claim 6, wherein the at least one therapeutically active substance is selected from the group comprising nucleic acids, amino acids, peptides, proteins, carbohydrates, lipids, glycoproteins, glycans or lipoproteins, wherein the aforementioned substances have antiproliferative, antimigration, antiangiogenic, antithrombotic, anti-inflammatory, antiphlogistic, cytostatic, cytotoxic, anticoagulative, antibacterial, antiviral and/or antimycotic properties.

9. Nanoparticle according to any one of the preceding claims, wherein the nanoparticle consists of superparamagnetic iron oxides or of pure iron having an oxide layer.

10. Nanoparticle according to any one of the preceding claims, wherein a sensitizer, radiosensitizer and/ or amplifier is bound to the nanoparticle for support of conventional cancer treatment methods.

11. Nanoparticle according to any one of the preceding claims, wherein monoclonal antibodies or antibody fragments and/ or aptamers, respectively, are coupled to the surface of the nanoparticle.

12. Infusion solution containing nanoparticles according to any one of claims 1 - 11.

13. Use of the nanoparticles according to any one of claims 1 - 11 for the preparation of a pharmaceutical composition for the treatment and/or prophylaxis of proliferative diseases, cancer and bacterial infections.

## Revendications

1. Nanoparticule, dans laquelle au moins une substance à action thérapeutique est liée à ladite nanoparticule par l'intermédiaire d'une molécule de liaison, dans laquelle la molécule de liaison contient un groupe thermolabile, labile électromagnétiquement, photolabile, labile en milieu acide, pouvant être intercalé ou clivable par voie enzymatique et dans laquelle la molécule de liaison est une molécule d'acide nucléique, un polypeptide, un acide nucléique peptidique, un aptamère, de l'ADN, de l'ARN, une glissière à leucine, un oligonucléotide, un oligopeptide, un pont anticorps-haptène ou un pont avidine-biotine, ou dans laquelle une liaison de biotine sur de l'avidine ou de la streptavidine est présente dans la molécule de liaison, et dans laquelle la séparation entre ladite au moins une substance à action thérapeutique et la nanoparticule est provoquée ou déclenchée ou essentiellement augmentée par un champ alternatif magnétique.

2. Nanoparticule selon la revendication 1, dans laquelle ladite au moins une substance à action thérapeutique est liée de manière covalente à la nanoparticule.

3. Nanoparticule selon l'une quelconque des revendications précédentes, dans laquelle la molécule d'acide nucléique est une construction d'acide nucléique double brin, une double hélice, un homo- ou hétéro-hybride d'ADN-ADN, d'ADN-ARN, d'ADN-ANP, d'ARN-ARN, d'ARN-ANP ou d'ANP-ANP.

4. Nanoparticule selon l'une quelconque des revendications précédentes, dans laquelle la nanoparticule est munie d'une enveloppe protectrice ou d'un revêtement.

5. Nanoparticule selon la revendication 4, dans laquelle l'enveloppe protectrice ou le revêtement présente des groupes amino ou des groupes carboxy.

6. Nanoparticule selon l'une quelconque des revendications précédentes, dans laquelle ladite au moins une substance à action thérapeutique est choisie dans le groupe comprenant des principes actifs antiprolifératifs, anti-migration, anti-angiogéniques, anti-thrombotiques, anti-inflammatoires, antiphlogistiques, cytostatiques, cytotoxiques, anticoagulants, antibactériens, antiviraux et/ou anti-mycosiques.

7. Nanoparticule selon la revendication 6, dans laquelle ladite au moins une substance à action thérapeutique est choisie dans le groupe comprenant l'actinomycine D, l'amétantrone, la 9-amino-camptothécine, l'aminoglutéthimide, l'amsacrine, l'anastrozole, les antagonistes de bases puriques et pyrimidiques, l'anthracycline, les inhibiteurs d'aromatase, l'asparaginase, les anti-oestrogènes, la bendamustine, le bexarotène, le biolimus A9, la bléomycine, la buséréline, le busulfan, la calichéamicine, la camptothécine, les dérivés de camptothécine, la capécitabine, le carboplatine, la carmustine, le chlorambucile, le cisplatine, la cladribine, le cyclophosphamide, la cytarabine, la cytosine arabinoside, les cytostatiques alkylés, la dacarbazine, la dactinomycine, la daunorubicine, la 5'-deoxy-5-fluorouridine, le docétaxel, la doxorubicine (adriamycine), la doxorubicine lipo, l'épirubicine, l'estramustine, l'étoposide, l'exemestan, la fludarabine, le fluorouracile, les antagonistes de l'acide folique, le formestane, la gemcitabine, les glucocorticoïdes, la goséréline, les hormones et les antagonistes d'hormones, l'hycamtine, l'hydroxyurée, l'idarubicine, l'iphosphamide, l'imatinib, l'irinotécan, le létrozol, la leuproréline, la lomustine, le maytansinoide, le melphalan, la mercaptopurine, le méthotrexate, la miltéfosine, la mitomycine, le mitopodozide, les inhibiteurs de mitose, la mitoxantrone, la nimustine, l'oxaliplatine, l'oxazaphosphorine, le paclitaxel, la pentostatine, les dérivés de la podophyllotoxine, la procarbazine, la rapamycine, la rhodomycine D, le tamoxifène, le témozolomide, le téniposide, la testolactone, le thiotépa, la thioguanine, les inhibiteurs de la topoisomérase, le topotécan, le tréosulfan, la trétinoïne, la triptoréline, le trophosphamide, les vinca-alcaloïdes, la vinblastine, la vincristine, la vindésine, la vinorelbine, les antibiotiques à action cytostatique.

8. Nanoparticule selon la revendication 6, dans laquelle la substance à action thérapeutique au moins au nombre de une est choisie dans le groupe comprenant les acides nucléiques, les acides aminés, les peptides, les protéines, les hydrates de carbone, les lipides, les glycoprotéines, les glycanes ou lipoprotéines, dans laquelle les substances susmentionnées présentent des propriétés antiprolifératives, anti-migration, antiangiogéniques, antithrombotiques, anti-inflammatoires, antiphlogistiques, cytostatiques, cytotoxiques, anticoagulantes, antibactériennes, antivirales et/ou antimycosiques.

9. Nanoparticule selon l'une quelconque des revendications précédentes, dans laquelle ladite nanoparticule est constituée d'oxydes de fer superparamagnétiques ou de fer pur comportant une couche d'oxyde.

10. Nanoparticule selon l'une quelconque des revendications précédentes, dans laquelle un sensibilisant, un radiosensibilisant et/ou un amplificateur servant à soutenir les méthodes de traitement du cancer conventionnelles est lié à la nanoparticule.

11. Nanoparticule selon l'une quelconque des revendications précédentes, dans laquelle des anticorps ou des fragments d'anticorps monoclonaux et/ou des aptamères sont couplés à la surface de ladite nanoparticule.

12. Solution pour perfusion contenant des nanoparticules selon l'une quelconque des revendications 1 à 11.

13. Utilisation des nanoparticules selon l'une quelconque des revendications 1 à 11 pour la production d'une composition pharmaceutique pour le traitement et/ou à la prophylaxie de maladies prolifératives, de cancers et d'infections bactériennes.
